# EUROPEAN PATENT APPLICATION

(11) **EP 3 181 242 A1**
(43) Date of publication of application: **21.06.2017**
(21) Application number: 15200496.6
(22) Date of filing: 16.12.2015
(51) Int. Cl.: B05B 17/06, B05B 5/08, A61L 2/22

(54) **ULTRASONIC FLUID DISPERSAL DEVICE**

(71) Applicant: IDFC AG, 9494 Schaan (LI)
(72) Inventor: ROBERTS, Stephen Mark, Troon, KA 10 6TA (GB); BATRAM, Mathias Joerg, D-82031 Gruenwald (DE)
(74) Representative: Kaminski Harmann

(57) **Abstract**

The invention pertains to an ultrasonic fluid dispersal device (1) comprising a tank unit (10) comprising a fluid inlet (12), a particle outlet (13), and a bottom region (15) that is adapted to hold an amount of fluid (50), wherein the fluid inlet (12) is adapted to supply the fluid (50) from a fluid reservoir (55) to the bottom region (15), and an ultrasonic transducer (20) that is arranged in the bottom region (15) and adapted to emit ultrasonic waves (29) into the fluid (50) causing at least a part of the fluid (50) in the bottom region (15) to be transformed into mist (57), characterized in that the transducer (20) covers at least two-thirds of a bottom side (11) of the tank unit (10), and the device (1) comprises a first air flow means (61, 62) which is adapted and arranged to create a first air flow (63, 64) to force dry particles (58) of the mist (57) through the particle outlet (13). The invention also pertains to a method for dispersing a fluid (50) comprising providing an amount of fluid (50) in a tank unit (10), emitting ultrasonic waves to the fluid (50) to generate mist (57), creating a first flow of air (63) to force dry particles (58) of the mist (57) towards and through a mist outlet (13) of the tank unit (10), and preventing wet particles (56) from the fluid (50) or the mist (57) to reach the mist outlet (13).

## Description

The present invention pertains to a fluid dispersal device comprising an ultrasonic transducer system. The fluid dispersal device is designed for atomizing fluids, particularly disinfectants and pesticides, and dispersing a dry output.

In traditional spraying methods for dispersing and coating surfaces with disinfectants and pesticides, the output is wet and comprises generally large chemical particles. Alternate methods for converting fluids to dry state chemicals are unreliable in their ability to output a genuinely dry product. Electro-static systems and foggers are extremely wet at close range.

It is known that fragrance, scents and other fluids can be dispersed into the air by cold air diffusion: Pressurised air is passed through a specially designed chamber in which it mixes with essential oils to create a fine vapour which is then dispersed into the surrounding air. The document WO 2005/105163 A1 discloses such a cold air dry diffusion device. Such devices are however incapable of producing sufficient chemical matter to deliver the required efficacy for pathogen or pest control.

Several problems arise due to the wetness of the output of known devices. Especially indoor use is prevented or severely limited when using such devices, as facilities need to dry out before they can be used again. Electronic and other wetness-sensitive items have to be removed from the location prior to treatment and replaced afterwards. If more than one coating of the same or a multitude of different chemicals must be provided, drying time between applications disadvantageously increases time and cost of treatment.

Furthermore, many of these treatment methods are expensive, inefficient and use more chemical product than necessary. They are also not reliable and often fail to coat the entire area as they rely on hand application making them regularly ineffective and inaccurate through human error.

The document WO 2008/015394 A1 discloses an ultrasonic atomiser for the dispersal of a variety of liquids. There is however only a very small output achievable with the disclosed setup.

Ultrasonic waves are nowadays used in various fields. Typical applications are ultrasonic washers, ultrasonic microphones and non-destructive testing. Ultrasonic scanning detectors are useful in medical electronics for clinical applications ranging from diagnosis to therapy and surgery. The sound source for the ultrasonic waves can be made from piezoelectric ceramics or from magneto-strictive materials, wherein hard piezoelectric ceramics are generally superior in efficiency and in size. It is known that ultrasonic transducers can be submerged in a rectangular tank of fluid to create a wet vapour fog, and these are regularly sold as garden pond fogging devices. These require a minimum level of fluid within the tank before the transducer will function as desired; this regularly amounts to in-excess of 300 to 2500 millilitres. It is desirable to reduce the size of the tank and the amount of fluid required within the tank to allow these devices to be practically portable and to require as little fluid as possible.

It is desirable to allow delivering chemicals, such as pest and pathogen control chemicals, in a dry format and without dependence on a human operator to direct the spray. It is moreover desirable to be able to deliver these chemicals in very small particle size as - particularly in pest control of crawling insects - the chemicals are more effective the smaller the particles are. In order to minimise the impact on revenue generating spaces, it is desirable to be able to deliver these particles quickly and in sufficient quantities. It is particularly desirable that more than 400 ml per hour of fluid can be converted to dry vapour matter in order to challenge pathogens and pests with sufficient efficacy and within a practical timeframe within a typical hotel room, healthcare treatment suite or shipping container of approximately 78 m³.

It is therefore an object of the present invention to provide an improved device and an improved method for dispersing a fluid.

Another object is to provide such a device and method wherein the output is dry.

It is another object of the present invention to provide such a device and method improving the coating of the particles on surfaces of the treated spaces.

At least one of these objects is achieved by the ultrasonic fluid dispersal device according to claim 1, the method according to claim 11, and/or the dependent claims of the present invention.

A first aspect of the present invention pertains to an ultrasonic fluid dispersal device that is adapted to disperse a fluid. Said device comprises a tank unit comprising a fluid inlet, a particle outlet, and a bottom region that is adapted to hold an amount of fluid, wherein the fluid inlet is adapted to supply the fluid from a fluid reservoir to the bottom region. The device moreover comprises an ultrasonic transducer that is arranged in the bottom region of the tank unit and is adapted to emit ultrasonic waves into the fluid causing at least a part of the fluid in the bottom region to be transformed into mist.

According to the invention, the transducer covers at least two-thirds of a bottom side of the tank unit, and the device comprises a first air flow means, e. g. a fan or an air compressor, which is adapted and arranged to create a first air flow to force dry particles of the mist through the particle outlet.

In one embodiment of the fluid dispersal device, for amplifying the ultrasonic waves created by the transducer, the tank unit is designed for superposition of the ultrasonic waves, in particular designed to create a standing wave in the fluid and/or a resonance chamber effect. In particular, the tank unit's walls are arranged to basically form a cylinder, a cone or a cone frustum, and/or are made from plastic or metal materials.

In another embodiment of the fluid dispersal device, the transducer covers at least 85% of the bottom side. Preferably, the transducer covers at least 95% of the bottom side or completely covers the bottom side, the bottom side preferably being basically circular.

In a further embodiment, the tank unit comprises a plurality of baffles that are arranged between the bottom region and the outlet, each baffle comprising an aperture, wherein the first air flow means is adapted and arranged to force the mist from the bottom region through the apertures, and the baffles and the apertures are adapted and arranged to prevent wet particles of the fluid from reaching the outlet and to enable dry particles to reach and pass the outlet to leave the tank unit.

Preferably, the tank unit comprises at least three - in particular at least four - baffles, the baffles being arranged with different distances to each other, particularly wherein the distances decrease with increasing distance to the bottom region.

The apertures preferably have different sizes - particularly wherein the sizes decrease with increasing distance to the bottom region - and are arranged so that there is no straight line between the outlet and the bottom region which is not blocked by at least one baffle.

The first air flow means (e. g. a fan or an air compressor) is preferably directed to or connected to the bottom region.

According to yet another embodiment of this aspect of the invention, the fluid dispersal device comprises an electrostatic charging module which is arranged at the particle outlet or downstream the particle outlet so that dry particles are driven through the electrostatic charging module, and adapted to electrostatic charge at least a part of the dry particles, so that electrostatic charged particles are dispersible into the environment that is to be treated, particularly a room.

In one embodiment, the electrostatic charging module is adapted to electrostatic charge a part of the dry particles by applying the electrostatic charging in a pulsed intermittent manner. For doing so, the electrostatic charging module may comprise a capacitor.

In another embodiment, the electrostatic charging module is adapted to apply the electrostatic charge with a plurality of different pulse frequencies. For selecting a pulse frequency, the device comprises at least one of the following:
- input means allowing a user to select from the plurality of pulse frequencies a pulse frequency or a sequence of pulse frequencies,
- input means allowing a user to input a room size of a room or other area to be treated, wherein a pulse frequency of the plurality of pulse frequencies is automatically selected based on the room size,
- means for determining a room size of a room or other area to be treated, wherein a pulse frequency of the plurality of pulse frequencies is automatically selected based on the determined room size, particularly wherein the means for determining the room size comprises means for measuring distances based on radar, lidar or sonar.

In another embodiment, the electrostatic charging module comprises an electrostatic chargeable mesh through which the dry particles are guidable. In this embodiment, the first air flow means particularly can be provided as a fan.

In a particular embodiment, a polarity of the electrically chargeable mesh can be changeable, particularly selectable by a user.

In yet another embodiment, the electrostatic charging module and/or the particle outlet has an orifice diameter of less than 10 millimetres, in particular less than 1.6 millimetres, wherein the first air flow means is an air compressor.

In a further embodiment, a second air flow means is provided which is adapted to create a second air flow to force the electrostatic charged mist away from the electrostatic charging module and into the environment or room.

Also said second air flow means may comprise a fan or an air compressor. Particularly, the second air flow means is adapted to provide at least two different ventilation modes. These differ at least by means of the strength of the second air flow. For selecting a ventilation mode, the device comprises at least one of the following:
- input means allowing a user to select a ventilation mode,
- means for determining a room size, wherein a ventilation mode is automatically selected based on the determined room size.

In another embodiment of the fluid dispersal device according to this aspect of the invention, a fluid level detector is provided which is adapted to detect a level of the fluid in the bottom region and to control a supply of the fluid from the fluid reservoir based on a detected level. Particularly, the fluid inlet is connected to a fluid pump, e. g. a peristaltic fluid pump, and the fluid level detector is adapted to control the pump based on the detected level. Additionally, the transducer can be adapted to adjust an intensity and/or frequency of the emitted ultrasonic waves based on a detected level.

In one embodiment, the tank unit comprises a fluid outlet, and the fluid level detector is adapted to control a draining of fluid out of the tank unit through the fluid outlet - particularly back into the fluid reservoir - based on the detected level.

In another embodiment, the tank unit is designed so that neither cables nor pipes transit the bottom side or a wall of the tank unit below a maximum fluid level in the bottom region. An insert unit can particularly be provided comprising the fluid inlet, the transducer and optionally further components, wherein the insert unit is designed so that it can be taken out from the tank unit by a user - e. g. for service and maintenance. The cables or pipes are then lead through a tube of the insert unit to a top end of the tank unit. Particularly, the tank unit comprises a fixing rail or a laterally attached tube for accepting the insert unit.

In yet another embodiment, the fluid dispersal device comprises accommodation means for accepting an exchangeable fluid reservoir, wherein the accommodation means comprises a feeder tube extension with a first feeder tube coupling, and is adapted to connect the exchangeable fluid reservoir to the fluid inlet so that the first feeder tube coupling couples with a second feeder tube coupling of a feeder tube of the exchangeable fluid reservoir. A pump is provided at the inlet or at the feeder tube extension to draw the fluid through the feeder tube and the feeder tube extension towards the fluid inlet.

Another aspect of the present invention pertains to a method for dispersing a fluid, particularly by means of a fluid dispersal device according to the first aspect of the invention. Said method comprises
- providing an amount of fluid in a tank unit,
- emitting ultrasonic waves to the fluid to generate mist,
- creating a first flow of air to force dry particles of the mist towards and through a particle outlet of the tank unit, and
- preventing wet particles from the fluid or the mist to reach the particle outlet.

In one embodiment, the method comprises an electrostatic charging of at least a part of the dry particles and releasing them as electrostatic charged particles into the environment, particularly into a room.

In another embodiment, the method comprises applying the electrostatic charging in a pulsed intermittent manner, particularly by means of a capacitor, and selecting a pulse rate for applying the electrostatic charging. In particular, the method comprises determining or inputting a size of the room, and the pulse rate is selected based on the size of the room.

In a further embodiment of the method, electrostatic charging of a part of the dry particles comprises guiding the particles through an electrically charged mesh, particularly wherein a polarity of the electrically charged mesh is user-selectable.

There are many applications for which the device and method according to the invention can be used. These include but are not limited to:
1. Infection prevention and control. This application comprises dispersing disinfectants into the air, onto surfaces, products and also onto hands.
2. Pest prevention and control. This application comprises dispersing pesticides into the air, onto surfaces, products and livestock within food production and animal husbandry.
3. Fragrancing. This application comprises dispersing pure essential oils and synthetic oils into the air.
4. Agriculture and food production. This application comprises dispersing nutrients and shelf life extending chemicals onto plants and food products
5. Restoration. This application comprises dispersing chemicals onto wooden surfaces for the purposes of combatting and treating wood weakening life forms.
6. Security. This application comprises dispersing chemicals onto products and people for the purposes of identification and tracking.

The business sectors in which infection as well as pest prevention and control can be applied include health, education, hotels, cruise ships, military, oil and gas operators, airlines, general transportation, and food production manufacture.

The business sectors for the application of fragrancing include hotels, retail, workplaces, commercial cleaning, and cruise ships.

For agriculture and food production, the business sectors comprise fruit farming, flowers, and packaging.

For restoration applications, the business sectors include flood and fire restoration, preventative treatment services, and museum and archive operators.

The business sectors for security applications include security business e. g. seeking to identify people at secure locations or crime scenes where a perimeter has been breached.

The invention in the following will be described in detail by referring to exemplary embodiments that are accompanied by figures, in which:
- Fig. 1: shows a first exemplary embodiment of a fluid dispersal device according to the invention;
- Fig. 2: illustrates the working principle of the fluid dispersal device of Fig. 1;
- Fig. 3: shows a second exemplary embodiment of a fluid dispersal device according to the invention;
- Fig. 4a: shows the accommodation means of the fluid dispersal device of Fig. 3;
- Fig. 4b: shows an exchangeable fluid reservoir for use with the fluid dispersal device of Fig. 3;
- Fig. 5: shows a third exemplary embodiment of a fluid dispersal device according to the invention;
- Fig. 6: illustrates the working principle of the fluid dispersal device of Fig. 5;
- Fig. 7: shows a fourth exemplary embodiment of a fluid dispersal device according to the invention; and
- Fig. 8: shows an exemplary embodiment of a fluid dispersal device according to the invention dispersing partially electrostatic charged mist into a room;
- Fig. 9: shows a fifth exemplary embodiment of a fluid dispersal device according to the invention;
- Figs. 10a-c: show a sixth exemplary embodiment of a fluid dispersal device according to the invention; and
- Fig. 11: shows a seventh exemplary embodiment of a fluid dispersal device according to the invention.

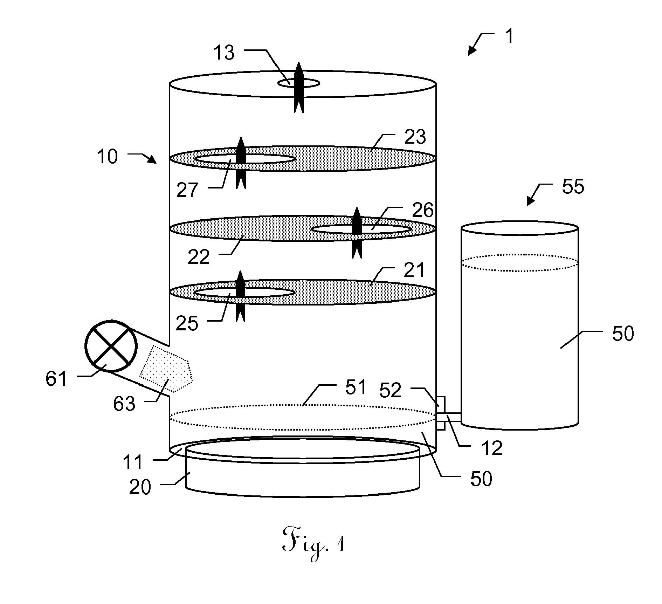

Figure 1 shows a first exemplary embodiment of a fluid dispersal device 1 according to the invention.

The device 1 comprises a basically cylindrical tank unit 10 that is adapted to hold an amount of fluid 50 in its bottom region. At the bottom side 11 of the tank unit 10, an ultrasonic transducer 20 is provided in such a way that it is submergible by the fluid 50.

Applicant has discovered that by changing the tank unit's shape from square or rectangular to cylindrical, it is possible to significantly reduce the minimum amount of fluid 50 required by 67%.

Furthermore, applicant has discovered that by restricting the fluid 50 to directly above the transducer 20, a more volatile effect is caused within the fluid 50 as a result of concentrating the shock waves from the transducer 20 in a more focused and contained area. Therefore, preferably, the transducer 20 covers the complete bottom side 11 of the tank unit 10 or nearly the complete bottom side 11, in particular more than 90% of the bottom side 11.

Preferably, the walls of the tank unit 10 are made out of plastic or metal materials and adapted to create a resonance chamber effect within the containing walls, further amplifying the sonic waves created by the transducer 20. This results in a higher output of conversion from fluid 50 to mist per hour of use. This increase is significant and represents a performance improvement of at least 50% and as high as 200% allowing for smaller transducers 20 to be used. Furthermore, applicant has discovered that with this increased performance, it becomes possible to reduce the voltage power to the device 1, creating less cavitation in the tank unit 10 which in turn reduces the moisture content in the mist created, while still converting sufficient mist significantly more quickly than a traditional arrangement would have allowed.

With the presented arrangement of the tank unit 10 it becomes possible to reduce the size of the device 1 considerably, and to create mist with considerably less moisture content. It also allows using smaller refill cartridges designed to hold a quantity of fluid that is calculated to contain the correct amount of fluid to treat a room of e. g. 78m³ rapidly. This total amount is only 40% of what would be the minimum fill level required within a small rectangular tank arrangement before the transducer would actually function, thus also reducing the device's requirement to constantly hold at least 300ml of fluid (which can become stale) as a minimum fluid level and which makes it impractical as a portable device. The reduced size also allows applications of the device where tank size is a limiting consideration - such as vehicle installations, portable systems, hand disinfection systems etc.

The fluid 50 is provided to the tank unit 10 from an external fluid reservoir 55 through an inlet 12 of the tank unit 10. The provision of fluid is controlled by fluid level sensor 52 which is adapted to determine a fluid level 51 and to hold the fluid level 51 at an optimum. The fluid reservoir 55 preferably is either exchangeable or refillable.

A fan 61 is arranged at the tank unit 10 to provide an air flow 63 in the tank unit 10 and to force mist out of an outlet 13 of the tank unit 10.

Within the upper section of the cylindrical tank unit 10, a series of baffles (in this example three baffles 21, 22, 23) are arranged between the fluid holding bottom section of the tank unit 10 and the outlet 13 at the top of the tank unit 10, each baffle having an aperture 25, 26, 27 allowing air and mist to pass the respective baffle.

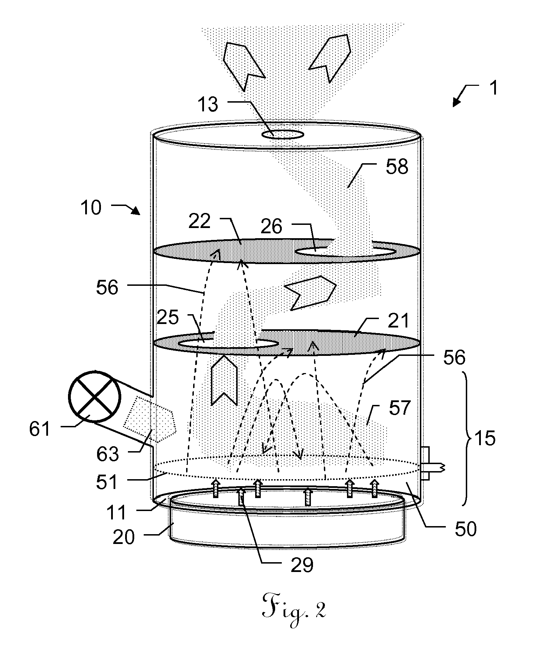

In Figure 2, the working principle of the tank unit 10 of the device 1 of Figure 1 is illustrated. It is to be noted that the fluid reservoir is not shown in this figure.

Fluid 50 is provided to the bottom part 15 of the tank unit 10 in such a way that the transducer 20 is completely covered by the fluid 50 and the fluid level 51 is at an optimum height above the transducer. The optimum height mainly depends on the used transducer 20 and on the kind of liquid 50 to be dispersed.

When supplied with electricity, the transducer 20 emits ultrasonic waves 29 into the fluid 50 that is positioned above the transducer 20. Oscillation energy of the transducer 20 spreads to the fluid's surface. If the surface is at an appropriate height above the transducer 20, the surface tension of the fluid 50 will be greatly reduced, dividing the fluid's surface into micro-particles, which causes a nebulizing of at least a part of the fluid 50, transforming this part of the fluid 50 into mist 57.

A resonance chamber effect is created within the containing walls of the tank unit 10, further amplifying the effect of the sonic waves created by the transducer 20.

The created mist 57 is then forced by the air flow 63 generated by the fan 61 from the bottom region 15 of the tank unit 10 through the apertures 25, 26 of the series of baffles (in this example comprising two baffles 21, 22).

Further, caused by the ultrasonic waves 29, some larger wet particles 57 of the fluid, e. g. droplets and splashes, are thrown upwards from the surface of the fluid 50. The baffles 21, 22 ensure that none of the droplets and splashes 56 is able to escape the bottom region 15 and that the output mist is dry. The resulting dry particles 58 ("dry mist") are then forced through the outlet 13 and into the surrounding air.

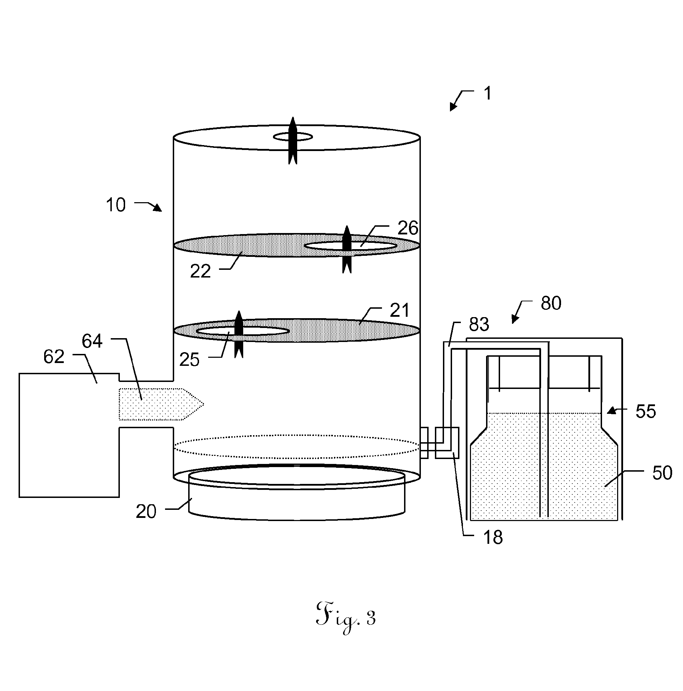

Figure 3 shows a second exemplary embodiment of a fluid dispersal device 1 according to the invention. This embodiment differs from the device of Figure 1 by having an air compressor 62 instead of a fan for creating a positive air pressure 64 to force the dry particles 58 out of the outlet 13.

A fluid pump 18 is provided at the inlet 12 to supply the necessary amount of fluid 50 to the tank unit 10. For example, the fluid pump can be a peristaltic pump. Advantageously, a fluid level and flow control system comprising the fluid level sensor 52 and the fluid pump 18 ensures that the required level 51 of fluid 50 is maintained within the tank unit 10 during operation.

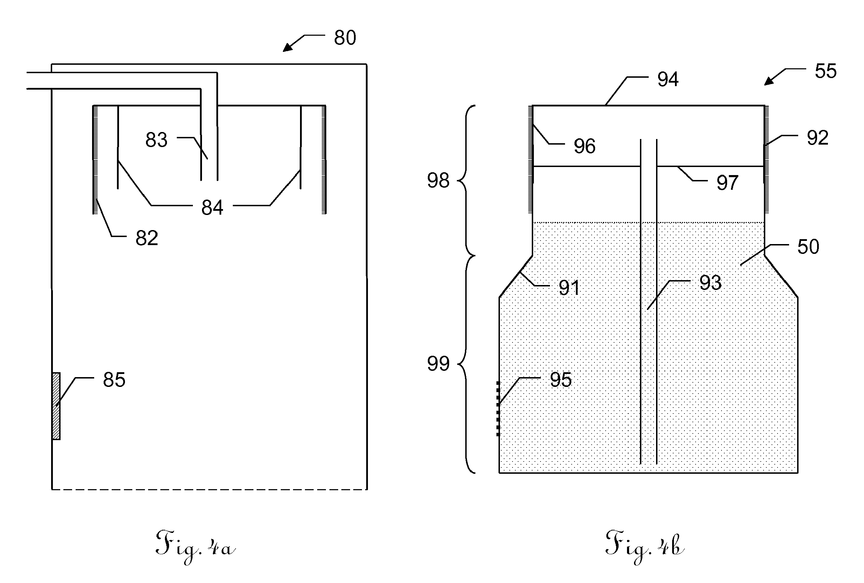

Moreover, the device 1 comprises a special accommodation means 80 for accepting an exchangeable fluid reservoir 55. Such accommodation means 80 and such a fluid reservoir 55 are described in the European patent application with the application number EP 15186753.8. Accommodation means 80 and fluid reservoir 55 are shown more detailed in the Figures 4a and 4b.

Figure 4a shows the accommodation means 80 of the device 1 of Figure 3 in a sectional view. The accommodation means 80 is provided inside of a housing that is adapted to also envelop a fluid reservoir when connected to the accommodation means 80. The accommodation means 80 comprises a screw thread 82 that is adapted to accept a screw thread of the fluid reservoir of Figure 4b so that the fluid reservoir is screwable onto the accommodation means 80. Alternatively, other fittings can be used for connecting the fluid reservoir to the accommodation means e. g. comprising a compression fit or a bayonet fit.

The accommodation means further comprise a feeder tube extension 83 which connects as a male part with a feeder tube of the fluid container as a female part in order to create a fluid tight connection between the feeder tube extension 83 and the feeder tube. Furthermore, in this embodiment a circular blade 84 is provided that is adapted to cut a sealing of the fluid reservoir when it is screwed onto the accommodation means 80 and before the feeder tube extension 83 couples with the feeder tube. A seal breaching means can of course also be provided with alternative fittings of the accommodation means.

The accommodation means' housing comprises sensor means 85 for reading a machine-readable code on the fluid reservoir, the code allowing the system to identify the fluid reservoir, or the fluid inside the reservoir, respectively. In this example, the sensor means 85 are embodied as a camera or a barcode scanner that is arranged and adapted to capture a printed code on the fluid reservoir. Preferably, the code can be provided as an ultraviolet barcode. Alternatively, other sensors can be used, e. g. an RFID (radio-frequency identification) reader can be provided that is adapted to read an RFID tag of the fluid reservoir. Also near field communication (NFC) devices and tags can be used. This sensor means 85 preferably interfaces with a control timer printed circuit board and processor system of the device and will not permit the device to operate until the sensor means 85 detects an original code that is within pre-programmed parameters, nor will it accept any code that it has previously been recognised and approved. Furthermore, the amount of fluid provided to the tank unit, an intensity or frequency of the ultrasonic waves, an intensity of the air flow or other parameters of the device can be controlled according to the kind of identified fluid 50.

Figure 4b shows the exchangeable fluid reservoir 55 to be used with the device 1 of Figure 3 in a sectional view, the fluid reservoir 55 being adapted to be accepted by the accommodation means 80 of Figure 4a. The fluid reservoir 55 has a body 91 made from glass, plastic, aluminium or steel and comprises a neck part 98 and a corpus part 99. The neck part 98 comprises a screw thread 92 that is adapted fit on the screw thread of the accommodation means so that the fluid container is screwable onto the accommodation means, and the corpus part 99 encloses a volume that accommodates the fluid 50. Alternatively, other fittings can be used for connecting the fluid reservoir to the accommodation means e. g. comprising a compression fit or a bayonet fit.

For instance, the fluid 50 can comprise insect repellents or insecticides, e. g. for repelling or killing mosquitoes or bed bugs. Alternatively, it can comprise air-dispersable fragrant, odour eliminator or disinfectant solutes or a combination thereof. The fluid 50 can comprise one or more essential oils or be a water based solution.

The fluid reservoir 55 comprises an insert having a feeder tube 93 and positioning means 97 adapted to fixedly position the feeder tube 93 in the fluid reservoir 55. The feeder tube 93 is adapted to connect with the feeder tube extension of Figure 4a. The fluid reservoir 55 also comprises a seal 94 that prevents the fluid 50 from leaving the fluid reservoir 55. It can be provided as a part of the insert or separately. The insert is connected to the body 91 of the fluid reservoir 55 by means of a ring-shaped surface 96 which contacts the inner surface of the neck part 98, preferably forming a liquid-tight connection with the neck part 98. The insert can be permanently fixed to the body 91, e. g. by means of bonding or gluing.

On the body 91, a machine-readable code 95 is provided that allows identification of the fluid reservoir 55 or its fluid 50, as described above. In this case, the code 95 is an ultraviolet barcode that is invisible to the human eye and positioned in such a way that the sensor of Figure 4a is able to read the encoded information. Preferably, the same barcode is printed on the body 91 at more than one position. Alternatively, the machine-readable code 95 can be provided by an RFID or NFC tag.

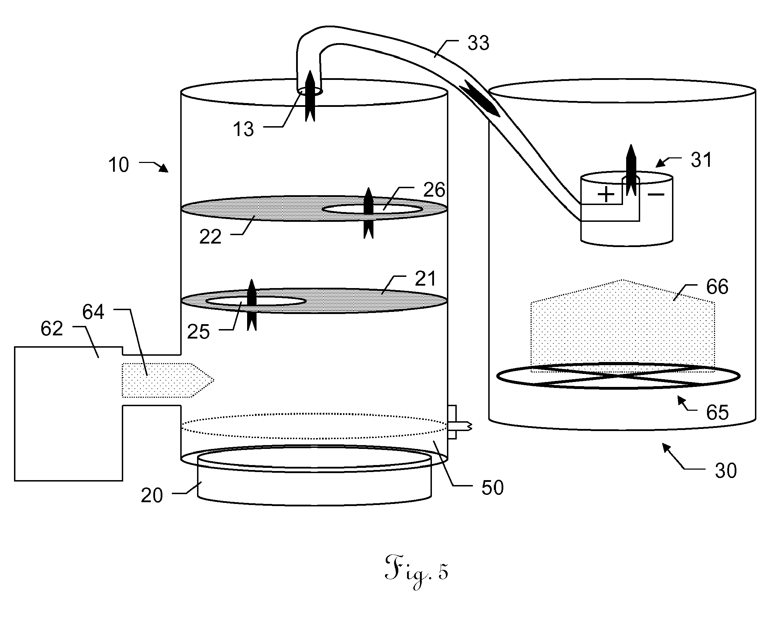

Figure 5 shows a third exemplary embodiment of a fluid dispersal device 1 according to the invention. The tank unit 10 is basically set up as in the second embodiment shown in Figure 3. The fluid reservoir is not depicted here. Additionally, this embodiment comprises a charging unit 30 to which dry particles are supplied from the tank unit 10 and from which electrostatic charged particles can be dispersed into the environment which is to be treated, e. g. into a room.

The charging unit 30 comprises an electrostatic charging module 31 which is arranged downstream the mist outlet 13 of the tank unit 10 so that dry particles are driven through the electrostatic charging module 31 before being dispersed. In this example, it is connected to the particle outlet 13 of the tank unit 10 by a tube 33.

Although the tank unit 10 and the charging unit 30 are shown here as separate units, they can obviously as well be provided in a common, e. g. cylindrical housing. The electrostatic charging module 31 can then be connected directly to the particle outlet 13, so that no or only a very short tube 33 is necessary.

The electrostatic charging module 31 is adapted to electrostatic charge at least a part of the dry particles which are driven through it in order to enhance the coating process of the particles on surfaces. Preferably, the electrostatic charge can be applied to the dry particles in a pulsed intermittent manner. The charging unit 30 in this embodiment also comprises a fan 65 that generates a flow of air 66 to drive the generated electrostatic charged particles away from the electrostatic charging module 31 and into the room or other environment.

This design allows for significant improvement in the device 1 with a reduction in the amount of fluid 50 required within the tank unit 10 and therefore a reduction in the overall size of the device 1 which is desirable in many instances.

It is difficult to cost-effectively and safely deliver an electrostatic charge effectively using traditional electrostatic methods with an outlet 13 size required for a fan based system shown in Figure 1, as the particle outlet orifice cannot be smaller than the fan. Otherwise backpressure would be created causing the system to fail. To be able to provide a small localised electrostatic charge in the desired manner, the orifice of the particle outlet 13 must be considerably smaller than 10 mm in diameter and preferably 1.6 mm or less. An air compressor 62 or similar means must be used rather than a fan to propel the transducer formed mist out from within the tank to be able to do so through such a small particle outlet 13 orifice.

The particle outlet 13 and the tube 33 have an orifice diameter of 1.6 mm, and the dry particles 57 are forced through this outlet 13 under a pressure (created by the air compressor 62) of about 4 psi (≈ 0.28 bar). Due to this small diameter it is possible to provide a small localised electrostatic charge to the dry particles 58.

The electrostatic charging module 31 is adapted to electrostatic charge a part of the dry particles 58 by applying the electrostatic charging in a pulsed intermittent manner. To do so, the electrostatic charging module 31 can optionally comprise a capacitor (not shown) to provide higher voltage with less energy consumption.

A pulse frequency or a voltage can optionally be adaptable - either by a user input, or automatically and based on various parameters, e. g. the kind of fluid 50 or the size of a room or other area to be treated.

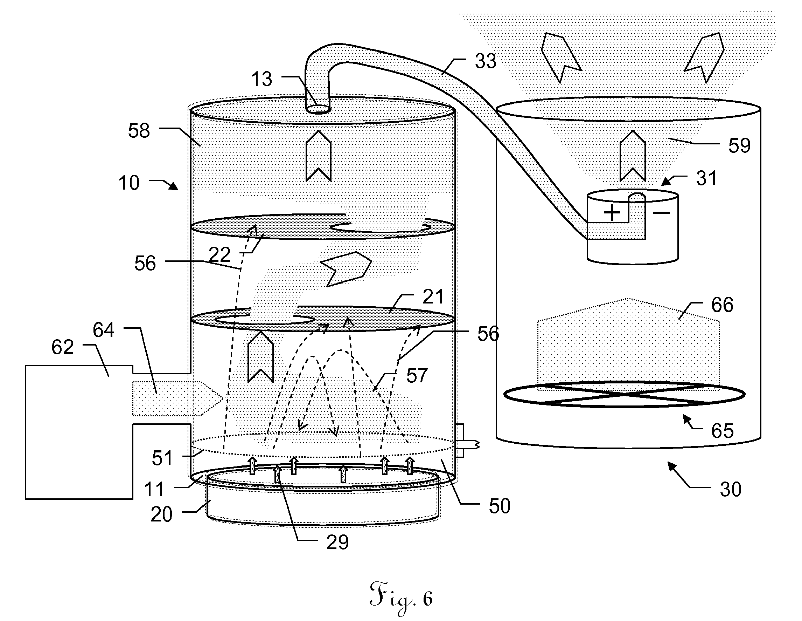

In Figure 6, the working principle of the device 1 of Figure 5 is illustrated.

As already described with respect to Figure 2, the transducer 20 emits ultrasonic waves 29, which causes a nebulizing of at least a part of the fluid 50 covering the transducer 20, transforming this part of the fluid 50 into mist 57.

This mist 57 is then forced by the air flow 64, or by the positive air pressure respectively, which is generated by the air compressor 62, from the bottom region 15 of the tank unit 10 through the apertures 25, 26 of the baffles 21, 22.

The number of baffles 21, 22 and the spacing between these can be varied in order to further reduce the particle size of the dry output 58. Moreover, the baffles 21, 22 ensure that none of the droplets and splashes 56 that are thrown upwards from the surface of the fluid 50 escape from the bottom region 15, so that the output mist is sufficiently dry for electrostatic charging. The resulting dry output 58 is then forced through the particle outlet 13 and the tube 33 connected thereto, arriving at the electrostatic charging module 31.

Due to the small orifice diameter of 1.6 mm, and the dry state of the mist, it becomes possible to electrostatic charge the dry particles 58 which pass the electrostatic charging module 31 in order to increase adhesion of the particles to surfaces.

The particles are charged in the charging module 31 in a pulsed intermittent manner. This means that only a part of the dispersed particles are actually charged, and the rest are dispersed without being electrostatic charged. This pulsed intermittent charging can be accomplished for instance by using a capacitor.

A pulse frequency for this pulsed intermittent charging can preferably be selected according to features of the room or other area into which the particles are to be dispersed. These features comprise the room's size, the number and spatial distribution of surfaces and their surface structure. For example, electrostatic charged particles tend to adhere to nearby surfaces, thus not being able to adhere to surfaces farther away. Selecting a suitable pulse frequency for a determined room size can thus ensure that all surfaces will be sufficiently coated by charged or non-charged mist. Additionally or alternatively, the pulse frequency can also depend on the kind of the fluid or on the kind of application. For instance, disinfectant mist may be desired to have more adhesion to surfaces than fragrancing mist, and insecticides against crawling insects require more adhesion to surfaces than insecticides against flying insects.

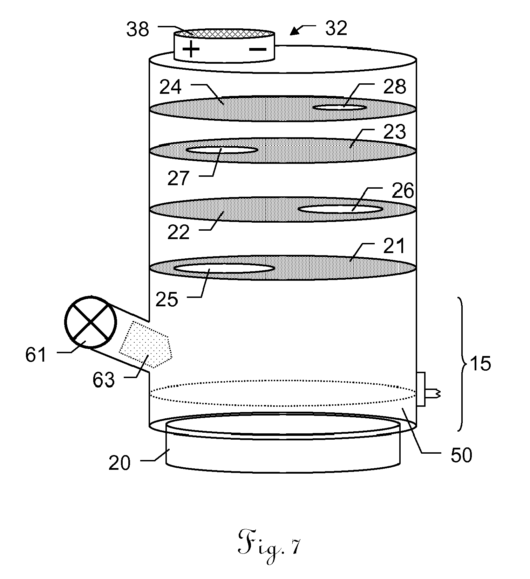

Figure 7 shows a fourth exemplary embodiment of a fluid dispersal device 1 according to the invention.

The depicted tank unit 10 has an alternative internal baffle structure which further ensures that only the smallest particles are ejected from the device. In this embodiment, four baffles 21-24 are arranged with decreasing spaces between them with increasing distance to the transducer 20. Their apertures 25-28 each have a different orifice size which likewise decreases with increasing distance to the transducer 20.

In some applications it is desirable to be able to electrostatically charge dry output at low pressure for the purposes of creating a charged dry output in a more localised area which does not travel as far due to its lower velocity.

The embodiment of Figure 7 shows therefore an alternative electrostatic charging module 32 and a device 1 which in contrast to the air compressor 62 operated system of Figure 5 works with a fan 61 as air flow means. As the orifice of the outlet cannot be reduced as much as it would be necessary to use the electrostatic charging module 31 of the system 1 of Figure 5, another setup for the electrostatic charging of the dry output is needed.

The electrostatic charging module 32 shown here is positioned directly on the particle outlet (not shown) and comprises an electrically charged mesh 38, through which the particles are driven. Compared to the device of Figure 5, the particle outlet orifice is wider, which is a requirement when using a fan 61 rather than compressed air to expel the dry matter created by the transducer 20 out of the particle outlet. The wire mesh electrostatic charging module 32 is positioned across the area of the wider particle outlet.

Another advantage of this embodiment is that through the use of the electrically charged mesh 38 it becomes possible to select the polarity of the electrostatic charging of the dry particles, i.e. between a positive and a negative charge. This advantageously increases particle adhesion to surfaces of opposite polarity. Optionally, both polarities can be applied sequentially to the mist flowing through the mesh 38, preferably with a phase without any charging in between.

The electrostatic charging modules 31, 32 described with respect to Figures 5, 6 and 7 can be used for other kinds of fluid disperser systems as well. In the European patent application with the serial number 15186753.8, a fluid dispersal system operating with cold air diffusion is described. When arranging an electrostatic charging module as described further above at the mist output vent or downstream the mist output vent of the fluid dispersal system of said application 15186753.8, electrostatic charged dispersion can be dispersed out of the system. Such a fluid dispersal system preferably comprises multiple diffusion chambers (or Venturi chambers) and advantageously can be designed as a man portable mobile device, i.e. being useable while being carried by the user, e. g. in a backpack.

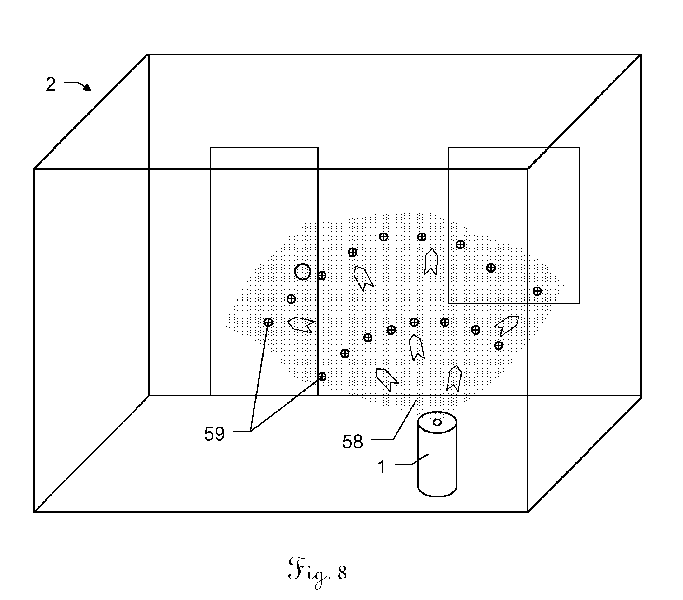

Figure 8 shows an ultrasonic fluid dispersal device 1 according to the invention that is positioned in a room 2 to disperse dry output 58 into this room 2. The output 58 comprises electrostatic charged parts 59. Due to its partially electrostatic charge, the coating of the room's surfaces with the dispersed particles 58 is enhanced.

Depending on the size of the room 2, the intensity of the electrostatic charging, in particular the percentage of the dispersed output which is charged, can be regulated. Here, the device 1 has electrostatic charged the particles 58 in an intermittent manner, so that charged parts 59 and uncharged parts of the output 58 are dispersed alternating. The pulse frequency can preferably be adapted to the size of the room 2 and/or to the distribution and kind of surfaces of the room 2. This might become necessary in order to arrive at an even distribution of the coating over the room's surfaces, as the charged part of the mist 59 tends to coat surfaces nearby the device 1 rather than surfaces that are farther away. In smaller rooms, more of the dispersed mist can therefore be charged, whereas with increasing room size, where some surfaces to be coated are farther away from the device 1, a larger percentage of the output 58 needs to be dispersed without being electrostatic charged.

In one embodiment, the device 1 can therefore comprise input means (not shown) allowing the user to input the size of the room 2 to be treated. For instance, this input means can comprise or be provided as a numeric keypad for inputting a floor area or a cubature of the room 2, a steplessly adjustable lever or a selection of switches for choosing between e. g. "open space" and a "large", "middle sized" or "small" room. Alternatively, the device 1 can comprise means for determining or estimating the room size (not shown), preferably automatically. For instance, this means can comprise distance measuring means for determining (approximate) distances to the room's surfaces, e. g. based on radar, lidar or sonar.

Although said "room" is depicted here as a room inside of a building, it can of course as well be any other three-dimensional space in which for treatment of its surfaces the ultrasonic fluid dispersal device 1 can be positioned, such as e. g. the interior of a vehicle, a container or a cave. The term "room size" therefore also stands for the size of other spaces.

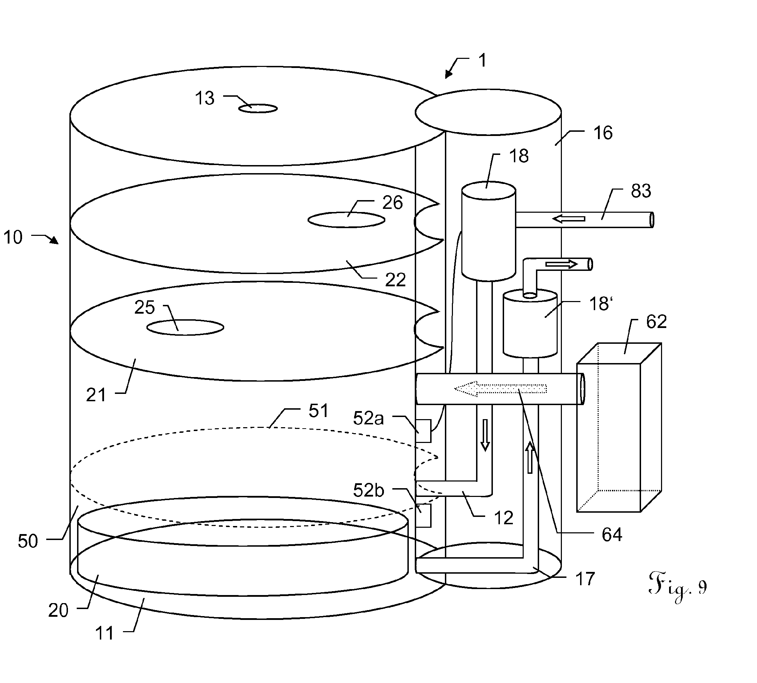

To ensure that the tank unit 10 of the device 1 is perfectly fluid tight, the tank unit 10 can preferably be designed so that neither cables nor pipes transit the bottom side or a wall of the tank unit below a maximum fluid level in the bottom region. Figure 9 shows a fifth exemplary embodiment of a fluid dispersal device 1 according to the invention. In this embodiment, the tank unit 10 comprises a smaller cylindrical side tube 16. All electricity and fluid supply lines are arranged in this side tube 16 in such a way that they exit the tank unit 10 above the fluid level 51 in the tank unit 10. Also the transducer 20 is fitted through the side tube 16 and therefore does not need to be fitted through the bottom side 11 of the fluid tank 10, allowing for the tank unit 10 to have guaranteed leak proof integrity.

Two baffles 21,22 are arranged between the bottom region holding the fluid 50 and the particle outlet 13. An air compressor 62 is provided for creating a positive air pressure 64 to force mist created by the transducer 20 out of the particle outlet 13.

A first fluid pump 18 is arranged above a maximum fluid level and adapted to pump fluid 50 from a fluid reservoir (not shown here), e. g. according to Figure 4b, through the feeder tube extension 83 to the fluid inlet 12. A second fluid pump 18' is adapted to pump fluid 50 out of the tank unit 10 through a fluid outlet 17.

A minimum and a maximum fluid level 51 for the transducer 20 to work properly are defined. The maximum fluid level also defines a line below which no cables or pipes transit a wall of the tank unit 10.

The fluid level sensor in this example is provided as an upper fluid level sensor 52a and a lower fluid level sensor 52b. It is adapted to control the fluid pumps 18, 18'. If the fluid level 51 is detected to be low (here by means of the lower fluid level sensor 52b), the first fluid pump 18 provides more fluid 50 from the fluid reservoir.

If the fluid level 51 is detected to be too high (here by means of the upper fluid level sensor 52a), excessive fluid 50 can be pumped out of the tank unit 10 through the fluid outlet 17, e. g. into the fluid reservoir. The same applies, if the device 1 is to be transported, or if the kind of fluid 50 is to be changed. For instance, based on a user command, the fluid 50 can completely be pumped out of the tank unit 10 and back into the exchangeable fluid reservoir, in order to prevent a spilling over of remaining fluid in the tank unit 10 during transport or an undesired mixing of two different fluids.

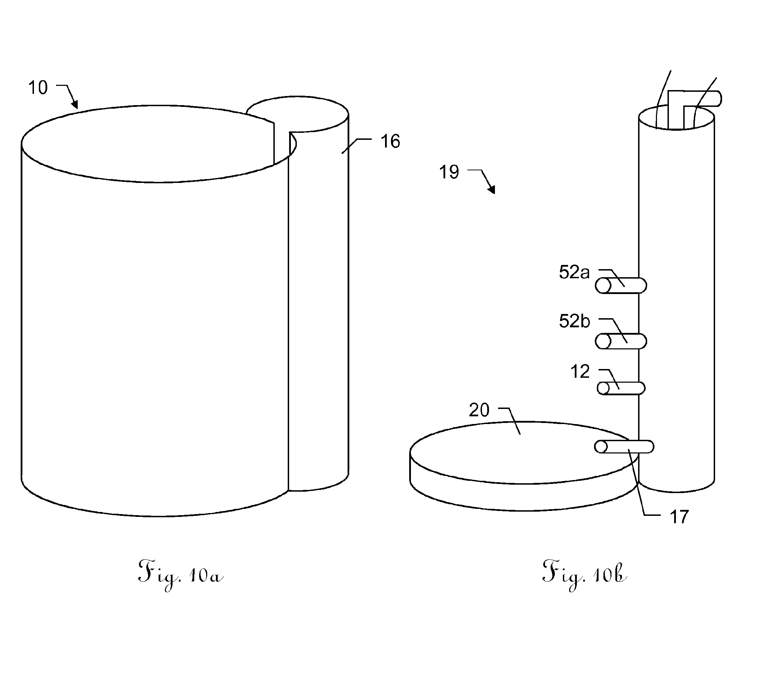

As shown in Figures 10a-c, the tube 16 and the transducer 20 can also be designed as an insert unit 19 that is introduced into the tank unit 10, so that the transducer 20 sits on top of the tank unit's bottom side 11. The services that are fixed inside this tube 16 are thus allowed to be slid down into the tank unit 10. All cables and pipes exit the insert unit 19 at the top. Optionally, also air pipes from ventilation means, such as a fan or an air compressor, can be lead through the tube of the insert 19 to provide an air flow or a positive air pressure to the tank unit 10.

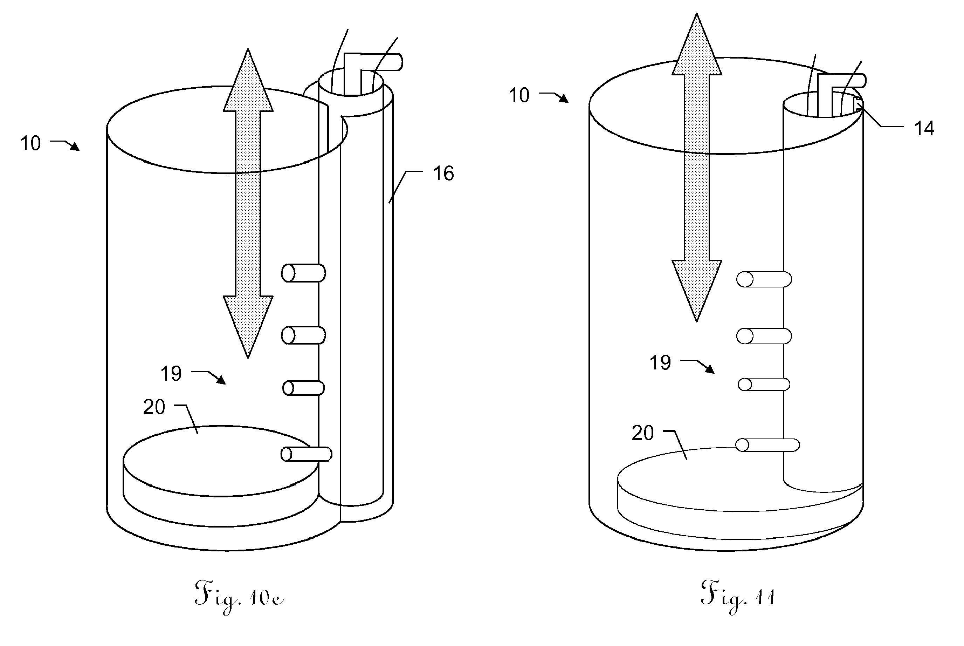

Figure 10a shows the tank unit 10 comprising a cylindrical main tank and a cylindrical side tube 16. The outer tank case is moulded in one piece including the bottom side 11, without any service penetrations. The slot-like side tube 16 allows the insert 19 shown in Figure 10b to slide down into it and where the various orifices and sensors protrude through the slot into the main body of the tank unit 10. A lid (not shown) can then be affixed atop. The tank unit 10 with the insert 19 inserted is shown in Figure 10c.

Another alternative setup is shown in Figure 11, where the insert unit 19 is placed directly in the tank unit 10. The tank unit 10 here has no side tube 16 to house the insert unit 19. Instead, the insert 19 is slid directly into the cylindrical tank unit 10. To be able to do so and to affix the insert 19 within the tank unit 10, the tank unit 10 comprises a fixing rail 14. The insert 19 has a female receiving slot on its rear to allow it to mate and affix to the male fixing rail 14. The transducer 20 is arranged as a part of the insert 19. Also baffles (not shown here) can be arranged as a part of the insert 19.

With the embodiments illustrated in Figures 10a-c and 11, a perfectly water tight tank unit 10 is achievable. Furthermore, it is possible to easily remove all technical components from the tank unit 10, which facilitates service, maintenance and cleaning of these components.

Although the invention is illustrated above, partly with reference to some preferred embodiments, it must be understood that numerous modifications and combinations of different features of the embodiments can be made. All of these modifications lie within the scope of the appended claims.

For instance, an arrangement of the tank unit according to Figure 9, 10c or 11 can be combined with the electrostatic charging modules of Figure 5 or Figure 7, and the accommodation means of Figure 3 can as well be used for the other described embodiments.

## Claims

1. Ultrasonic fluid dispersal device (1) comprising
- a tank unit (10) comprising a fluid inlet (12), a particle outlet (13), and a bottom region (15) that is adapted to hold an amount of fluid (50), wherein the fluid inlet (12) is adapted to supply the fluid (50) from a fluid reservoir (55) to the bottom region (15); and
- an ultrasonic transducer (20) that is arranged in the bottom region (15) and adapted to emit ultrasonic waves (29) into the fluid (50) causing at least a part of the fluid (50) in the bottom region (15) to be transformed into mist (57),
**characterized in that**
- the transducer (20) covers at least two-thirds of a bottom side (11) of the tank unit (10), and
- the device (1) comprises a first air flow means (61, 62) which is adapted and arranged to create a first air flow (63, 64) to force dry particles (58) of the mist (57) through the particle outlet (13).

2. Fluid dispersal device (1) according to claim 1,
**characterized in that**
- for amplifying the ultrasonic waves (29) created by the transducer (20), the tank unit (10) is designed for superposition of the ultrasonic waves (29), in particular designed to create a standing wave in the fluid (50) and/or a resonance chamber effect, particularly wherein the tank unit's walls
- are arranged to basically form a cylinder, a cone or a cone frustum, and/or
- are made from plastic or metal materials; and/or
- the transducer (20) covers at least 85% of the bottom side (11), particularly wherein
- the transducer (20) covers at least 95% of the bottom side (11) or completely covers the bottom side (11), and/or
- the bottom side (11) is basically circular.

3. Fluid dispersal device (1) according to any one of the preceding claims,
**characterized in that**
the tank unit (10) comprises a plurality of baffles (21-24) that are arranged between the bottom region (15) and the particle outlet (13), each baffle comprising an aperture (25-28), wherein
- the first air flow means (61, 62) is adapted and arranged to force the mist (57) from the bottom region (15) through the apertures (25-28); and
- the baffles (21-24) and the apertures (25-28) are adapted and arranged to prevent wet particles (56) of the fluid (50) from reaching the particle outlet (13) and to enable dry particles (58) to reach and pass the particle outlet (13) to leave the tank unit (10),
particularly wherein
- the tank unit (10) comprises at least three, in particular at least four, baffles (21-24);
- the baffles (21-24) are arranged with different distances to each other, particularly wherein the distances decrease with increasing distance to the bottom region (15);
- the apertures (25-28) have different sizes, particularly wherein the sizes decrease with increasing distance to the bottom region (15);
- the apertures (25-28) are arranged so that there is no straight line between the particle outlet (13) and the bottom region (15) which is not blocked by at least one baffle (21-24); and/or
- the first air flow means (61, 62) is directed to or connected to the bottom region (15).

4. Fluid dispersal device (1) according to any one of the preceding claims,
**characterized by**
an electrostatic charging module (31, 32) which is
- arranged at or downstream the particle outlet (13) so that dry particles (58) are driven through the electrostatic charging module (31, 32), and
- adapted to electrostatic charge at least a part of the dry particles (58),
wherein electrostatic charged particles (59) are dispersible into an environment, particularly into a room (2).

5. Fluid dispersal device (1) according to claim 4,
**characterized in that**
the electrostatic charging module (31, 32) is adapted to electrostatic charge a part of the dry particles (58) by applying the electrostatic charging in a pulsed intermittent manner, particularly wherein the electrostatic charging module (31, 32) comprises a capacitor,
in particular wherein the electrostatic charging module (31, 32) is adapted to apply the electrostatic charge with a plurality of different pulse frequencies, the device (1) comprising
- input means allowing a user to select from the plurality of pulse frequencies a pulse frequency or a sequence of pulse frequencies,
- input means allowing a user to input a size of the room (2) or other area to be treated, wherein a pulse frequency of the plurality of pulse frequencies is automatically selected based on the size of the room (2) or other area to be treated, and/or
- means for determining a size of the room (2) or other area to be treated, wherein a pulse frequency of the plurality of pulse frequencies is automatically selected based on the determined size of the room (2) or other area to be treated, particularly wherein the means for determining the size of the room (2) or other area to be treated comprises means for measuring distances based on radar, lidar or sonar.

6. Fluid dispersal device (1) according to claim 4 or claim 5,
**characterized in that**
the electrostatic charging module (32) comprises an electrostatic chargeable mesh (38) through which the dry particles (58) are guidable, particularly wherein a polarity of the electrically chargeable mesh (38) is user-selectable and/or the first air flow means is a fan (61).

7. Fluid dispersal device (1) according to claim 4 or claim 5,
**characterized in that**
the electrostatic charging module (31) and/or the particle outlet (13) have an orifice diameter of less than 10 mm, in particular less than 1.6 mm, wherein the first air flow means is an air compressor (62).

8. Fluid dispersal device (1) according to any one of claims 4 to 7,
**characterized by**
a second air flow means (65) adapted to create a second air flow (66) to force the electrostatic charged particles (59) away from the electrostatic charging module (31), particularly wherein
- the second air flow means (65) comprises a fan or an air compressor to blow the electrostatic charged mist (59) into a room (2) or other area to be treated, and/or
- the second air flow means (65) is adapted to provide at least two ventilation modes, which differ at least by a strength of the second air flow (66), wherein the system (1) comprises
- input means allowing a user to select a ventilation mode, and/or
- means for determining a size of the room (2) or other area to be treated, wherein a ventilation mode is automatically selected based on the determined size of the room (2) or other area to be treated.

9. Fluid dispersal device (1) according to any one of the preceding claims,
**characterized by**
a fluid level detector (52) which is adapted to detect a level (51) of the fluid (50) in the bottom region (15) and to control a supply of the fluid (50) from the fluid reservoir (55) based on a detected level (51), particularly wherein
- the fluid inlet (12) is connected to a fluid pump (18), and the fluid level detector (52) is adapted to control the pump (18) based on the detected level (51);
- the transducer (20) is adapted to adjust an intensity and/or frequency of the emitted ultrasonic waves based on a detected level (51); and/or
- the tank unit (10) comprises a fluid outlet (17), and the fluid level detector (52) is adapted to control a draining of fluid (50) out of the tank unit (10) through the fluid outlet (17), particularly into the fluid reservoir (55), based on the detected level (51).

10. Fluid dispersal device (1) according to any one of the preceding claims,
**characterized in that**
- the tank unit (10) is designed so that neither cables nor pipes transit the bottom side (11) or a wall of the tank unit (10) below a maximum fluid level in the bottom region (15); and/or
- the fluid inlet (12) and the transducer (20) are adapted as a part of an insert unit (19) which is designed to be withdrawable from the tank unit (10), particularly wherein the tank unit (10) comprises a fixing rail (14) or a laterally attached tube (16) for accepting the insert unit (19).

11. Fluid dispersal device (1) according to any one of the preceding claims,
**characterized by**
accommodation means (80) for accepting an exchangeable fluid reservoir (55), wherein the accommodation means (80) comprises a feeder tube extension (83) with a first feeder tube coupling, and is adapted to connect the exchangeable fluid reservoir (55) to the fluid inlet (12) so that the first feeder tube coupling couples with a second feeder tube coupling of a feeder tube (93) of the exchangeable fluid reservoir (55), wherein a pump (18) is provided at the inlet (12) or at the feeder tube extension (83) to draw the fluid (50) through the feeder tube (93) and the feeder tube extension (83) towards the fluid inlet (12).

12. Method for dispersing a fluid (50), particularly by means of a fluid dispersal device (1) according to any one of the preceding claims, the method comprising
- providing an amount of fluid (50) in a tank unit (10);
- emitting ultrasonic waves to the fluid (50) to generate mist (57);
- creating a first flow of air (63, 64) to force dry particles (58) of the mist (57) towards and through a particle outlet (13) of the tank unit (10); and
- preventing wet particles (56) from the fluid (50) or the mist (57) to reach the particle outlet (13).

13. Method according to claim 12,
**characterized by**
electrostatic charging of at least a part of the dry particles (58) and releasing them as electrostatic charged particles (59) into an environment, particularly into a room (2).

14. Method according to claim 13,
**characterized by**
applying the electrostatic charging in a pulsed intermittent manner, particularly by means of a capacitor, wherein the method further comprises selecting a pulse rate for applying the electrostatic charging, particularly wherein
- the electrostatic charged mist (59) is released into a room (2) or other area to be treated,
- the method comprises determining or inputting a size of the room (2) or other area to be treated, and
- the pulse rate is selected based on the size of the room (2) or other area to be treated.

15. Method according to claim 13 or claim 14,
**characterized in that**
the electrostatic charging of the dry particles (58) comprises guiding the particles through an electrically charged mesh (38), particularly wherein a polarity of the electrically charged mesh (38) is user-selectable.
